(19) Europäisches Patentamt
European Patent Office
Office européen des brevets

(11) **EP 4 092 122 A1**

(12) **EUROPEAN PATENT APPLICATION**
published in accordance with Art. 153(4) EPC

(43) Date of publication:
**23.11.2022 Bulletin 2022/47**

(21) Application number: **21779030.2**

(22) Date of filing: **18.02.2021**

(51) International Patent Classification (IPC):
**C12N 15/55** (2006.01)   **C12N 15/63** (2006.01)
**C12Q 1/34** (2006.01)   **C12Q 1/6813** (2018.01)

(52) Cooperative Patent Classification (CPC):
**C12N 9/14; C12N 15/63; C12Q 1/34; C12Q 1/68;
C12Q 1/6813**

(86) International application number:
**PCT/JP2021/006198**

(87) International publication number:
**WO 2021/199770 (07.10.2021 Gazette 2021/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.04.2020 JP 2020066984**

(71) Applicants:
• **TOPPAN INC.**
**Tokyo 110-0016 (JP)**
• **Kyushu University, National University
Corporation**
**Nishi-ku**
**Fukuoka-shi**
**Fukuoka 819-0395 (JP)**

(72) Inventors:
• **MAKINO Yoichi**
**Tokyo 110-0016 (JP)**
• **ISHINO Yoshizumi**
**Fukuoka-shi, Fukuoka 819-0395 (JP)**
• **ISHINO Sonoko**
**Fukuoka-shi, Fukuoka 819-0395 (JP)**

(74) Representative: **TBK**
**Bavariaring 4-6**
**80336 München (DE)**

Remarks:
The complete document including Reference
Table(s) and the Sequence Listing(s) can be
downloaded from the EPO website

(54) **METHOD FOR DETECTING TARGET NUCLEIC ACID**

(57) The present invention relates to a method for detecting a target nucleic acid, the method including cleaving a first flap of a first cleavage structure formed by a target nucleic acid, a first nucleic acid, and a second nucleic acid; cleaving a second flap of a second cleavage structure formed by a third nucleic acid, the cleaved first flap, and a fourth nucleic acid; and detecting the presence of the target nucleic acid by detecting the cleaved second flap, wherein cleaving the first flap and cleaving the second flap are carried out by cleaving the first flap and the second flap with a flap endonuclease, and the flap endonuclease has an amino acid sequence having a sequence identity of 96% or higher with an amino acid sequence of a flap endonuclease of Thermococcus kodakarensis strain KOD1, or the like.

Fig.2

**Description**

**Technical Field**

The present invention relates to a method for detecting a target nucleic acid.

**Background Art**

Various diagnostic methods based on the detection of target nucleic acids are known. Examples of such diagnostic methods include constitutional diagnosis by single nucleotide polymorphism (SNP) analysis, predictive diagnosis of the effects and side effects of a drug (for example, anticancer agent) by somatic cell mutation analysis, and diagnosis of infectious diseases by detection of viral DNA or RNA. In these diagnostic applications, since the abundance ratio of the target nucleic acid is often low, a method capable of quantitatively analyzing the target nucleic acid with high precision is required. As a technique for quantitatively analyzing a target nucleic acid with high precision, for example, methods of utilizing digital measurement are known.

Digital measurement is a quantitative method of diluting biomolecules such as target nucleic acids to be probabilistically one molecule or less, distributing the biomolecules into a large number of minute reaction vessels, binarizing (a signal associated with the detection reaction is present or absent) each of the reaction vessels, and thereby counting whether a detection target is contained in the reaction vessel. In the digital measurement, it is possible to detect a single biomolecule, and high-precision quantitative measurement can be performed. For example, Non Patent Literature 1 discloses a method for detecting a target nucleic acid by combining an ICA (Invasive Cleavage Assay) method and digital measurement.

**Citation List**

**Non Patent Literature**

Non Patent Literature 1: Journal of Printing Science and Technology, 2017, Vol. 54, No. 6, pp. 377-382.

**Summary of Invention**

**Technical Problem**

Digital measurement is based on binarization (a signal associated with the detection reaction is present or absent) of each reaction vessel, and therefore, in order to perform quantitative measurement with higher precision, an enhancement of the signal/noise ratio (S/N ratio) is required. Thus, it is an object of the present invention to provide a method for detecting a target nucleic acid with an enhanced S/N ratio.

**Solution to Problem**

The present invention relates to a method for detecting a target nucleic acid, the method including: cleaving a first flap of a first cleavage structure formed by a target nucleic acid, a first nucleic acid, and a second nucleic acid; cleaving a second flap of a second cleavage structure formed by a third nucleic acid, the cleaved first flap, and a fourth nucleic acid; and detecting the presence of the target nucleic acid by detecting the cleaved second flap, wherein the cleaving the first flap and the cleaving the second flap are carried out by cleaving the first flap and the second flap with a flap endonuclease, and the flap endonuclease has an amino acid sequence having a sequence identity of 96% or higher with an amino acid sequence of a flap endonuclease of a microbe selected from the group consisting of Thermococcus kodakarensis strain KOD1, Pyrococcus abyssi strain GE5, and Methanothermobacter thermautotrophicus strain Delta H.

In the detection method according to the present invention, since the cleaving the first flap and the cleaving the second flap are carried out by means of a specific flap endonuclease, the S/N ratio is enhanced as compared to conventional methods.

With regard to the above-described detection method, the target nucleic acid may be a DNA.

With regard to the above-described detection method, it is preferable that the cleaving the first flap and the cleaving the second flap are carried out under the conditions of a pH of 7.5 or higher and 9.0 or lower. When the pH is within this range, the effect of enhancing the S/N ratio becomes more remarkable.

With regard to the above-described detection method, it is preferable that the cleaving the first flap and the cleaving the second flap are carried out under the conditions of a temperature of 55°C or higher and 70°C or lower.

When the temperature is within this range, the effect of enhancing the S/N ratio becomes more remarkable.

With regard to the above-described detection method, it is preferable that the cleaving the first flap and the cleaving the second flap are carried out in an aqueous solvent, and that the aqueous solvent includes $Mg^{2+}$. Furthermore, it is more preferable that the concentration of $Mg^{2+}$ in the aqueous solvent is 2.5 mM or greater and 20 mM or less. As a result, the effect of enhancing the S/N ratio becomes more remarkable.

With regard to the above-described detection method, it is preferable that the cleaving the first flap and the cleaving the second flap are carried out in an aqueous solvent, and that the concentration of $K^+$ in the aqueous solvent is 0 mM or greater and 100 mM or less. As a result, the effect of enhancing the S/N ratio becomes more remarkable.

With regard to the above-described detection method, it is preferable that the cleaving the first flap and the cleaving the second flap are carried out in an aqueous solvent, and that the concentration of trishydroxymethylaminomethane in the aqueous solvent is greater than 0 mM and 300 mM or less. As a result, the effect of enhancing the S/N ratio becomes more remarkable.

With regard to the above-described detection method, the cleaving the first flap and the cleaving the second flap may be carried out in an aqueous solvent, and the concentration of the flap endonuclease in the aqueous solvent may be 0.5 $\mu$M or greater and 8 $\mu$M or less. As a result, a sufficient effect of enhancing the S/N ratio can be achieved.

With regard to the above-described detection method, it is preferable that the cleaving the first flap and the cleaving the second flap are carried out in an aqueous solvent, that the volume of the aqueous solvent is 10 aL or more and 10 $\mu$L or less. As a result, the effect of enhancing the S/N ratio becomes more remarkable.

With regard to the above-described detection method, the third nucleic acid and the fourth nucleic acid may be bonded by a linker molecule.

In the above-described detection method, the cleaved second flap may be detected by detecting fluorescence.

The present invention also relates to a kit for use in the above-mentioned detection method according to the present invention, the kit including the third nucleic acid, the fourth nucleic acid, the flap endonuclease, and a manual indicating the guidelines for designing the nucleotide sequences of the first nucleic acid and the second nucleic acid according to the nucleotide sequence of the target nucleic acid.

The present invention further relates to an expression vector having: a nucleic acid sequence that encodes an amino acid sequence having a sequence identity of 96% or higher with an amino acid sequence of a flap endonuclease of a microbe selected from the group consisting of Thermococcus kodakarensis strain KOD1, Pyrococcus abyssi strain GE5, and Methanothermobacter thermautotrophicus strain Delta H; and one or a plurality of regulatory sequences operably linked to the nucleic acid sequence.

The above-described expression vector can be used to prepare a flap endonuclease that can be suitably used for the above-described detection method according to the present invention. Furthermore, the above-described expression vector may be a plasmid.

## Advantageous Effects of Invention

According to the present invention, a method for detecting a target nucleic acid with an enhanced S/N ratio can be provided.

## Brief Description of Drawings

FIG. 1 is an explanatory diagram of a cleavage structure.
FIG. 2 is an explanatory diagram showing the outline of an evaluation system of Test Example 3.
FIG. 3 is a graph showing an example of the results of real-time measurement of the fluorescence intensity in Test Example 3.
FIG. 4 is a graph showing the results of real-time measurement of the fluorescence intensity in Test Example 4.
FIG. 5 is a graph showing the results of real-time measurement of the fluorescence intensity in Test Example 4.
FIG. 6 is a graph showing the results of real-time measurement of the fluorescence intensity in Test Example 4.
FIG. 7 is a graph showing the results of real-time measurement of the fluorescence intensity in Test Example 4.
FIG. 8 is a graph showing the results of real-time measurement of the fluorescence intensity in Test Example 4.
FIG. 9 is a graph showing the results of real-time measurement of the fluorescence intensity in Test Example 4.
FIG. 10 is a graph showing the results of real-time measurement of the fluorescence intensity in Test Example 4.
FIG. 11 is a graph showing the results of real-time measurement of the fluorescence intensity in Test Example 4.
FIG. 12 is a graph showing the results of real-time measurement of the fluorescence intensity in Test Example 4.
FIG. 13 is a graph showing the results of real-time measurement of the fluorescence intensity in Test Example 4.
FIG. 14 is a photograph showing the results of digital measurement in Test Example 5.

**Description of Embodiments**

Hereinafter, embodiments for carrying out the present invention will be described in detail. However, the present invention is not intended to be limited to the following embodiments.

The method for detecting a target nucleic acid according to the present embodiment includes: cleaving a first flap of a first cleavage structure formed by a target nucleic acid, a first nucleic acid, and a second nucleic acid (first flap cleaving step); cleaving a second flap of a second cleavage structure formed by a third nucleic acid, the cleaved first flap, and a fourth nucleic acid (second flap cleaving step); and detecting the presence of the target nucleic acid by detecting the cleaved second flap (detection step). Here, the cleaving the first flap and the cleaving the second flap are carried out by means of a specific flap endonuclease.

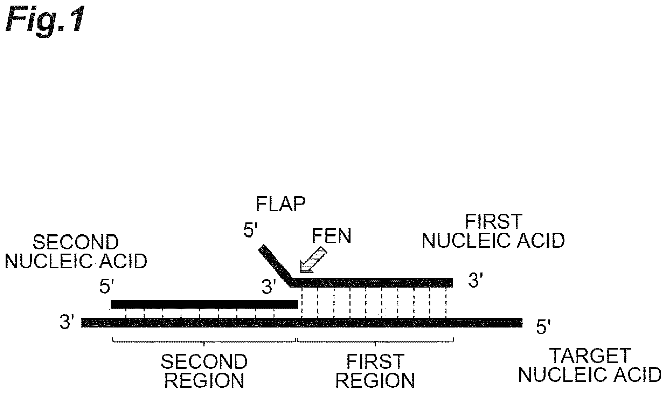

The "cleavage structure" according to the present specification is a structure formed of a target nucleic acid and two nucleic acids that can be hybridized to a first region and a second region, respectively, which are adjacent to the target nucleic acid, and means a structure in which a partial sequence on the 3' side of one nucleic acid (for example, first nucleic acid) hybridizes to the first region of the target nucleic acid, the other nucleic acid (for example, second nucleic acid) hybridizes to the second region adjacent to the 3' side of the first region of the target nucleic acid, and the remaining sequence on the 5' side of the nucleic acid hybridizing to the first region (for example, first nucleic acid) protrudes at one strand and forms a flap. A specific example of the cleavage structure may be the structure shown in FIG. 1.

The flap endonuclease (hereinafter, also described as "FEN" or "FEN protein") used for the detection method according to the present embodiment may be a flap endonuclease that has an amino acid sequence having a sequence identity of 96% or higher with the amino acid sequence of a flap endonuclease of a microbe selected from the group consisting of Thermococcus kodakarensis strain KOD1, Pyrococcus abyssi strain GE5, and Methanothermobacter thermautotrophicus strain Delta H. By using such a FEN, an enhanced S/N ratio as compared to that of conventional methods can be achieved.

The FEN used for the detection method according to the present embodiment may be one having an activity of recognizing a cleavage structure, cleaving the root of a flap (phosphodiester bond on the 3' side of the site where three nucleic acids overlap), and releasing the flap (nucleic acid).

Incidentally, Thermococcus kodakarensis strain KOD1, Pyrococcus abyssi strain GE5, Methanothermobacter thermautotrophicus strain Delta H is available from the RIKEN Bioresource Research Center (RIKEN BRC).

The FEN used for the detection method according to the present embodiment may be such that the amino acid sequence thereof has a sequence identity of 96% or higher with the amino acid sequence of a FEN possessed by the above-mentioned microbial strain. Furthermore, this FEN is one having an activity of recognizing a cleavage structure, cleaving the root of a flap (phosphodiester bond on the 3' side of the site where three nucleic acids overlap), and releasing the flap (nucleic acid).

According to the present specification, the term "sequence identity" means the proportion (%) of identical amino acid residues with respect to the overlapping complete amino acid sequence in an optimal alignment when two amino acid sequences are aligned by using a mathematical algorithm well known in the pertinent art (preferably, in this algorithm, introduction of a gap into one or both of the sequences can be considered for optimal alignment). For the creation of the alignment, for example, NCBI BLAST (National Center for Biotechnology Information Basic Local Alignment Search Tool) can be used.

With regard to the FEN used for the detection method according to the present embodiment, the sequence identity of the amino acid sequence thereof with the amino acid sequence of a FEN possessed by the above-mentioned microbial strain may be 96% or higher, may be 97% or higher, may be 98% or higher, may be 99% or higher, may be 99.1% or higher, may be 99.2% or higher, may be 99.3% or higher, may be 99.4% or higher, may be 99.5% or higher, may be 99.6% or higher, may be 99.7% or higher, may be 99.8% or higher, may be 99.9% or higher, or may be 100%.

The FEN protein used for the detection method according to the present embodiment may be a protein isolated by being extracted or purified from the above-mentioned microbial strain or may be a protein obtained by cloning a gene encoding the FEN protein from the above-mentioned microbial strain, causing the gene to be expressed in a heterologous protein expression system (recombinant protein expression system), and isolating the expression protein by extraction or purification. Furthermore, the FEN protein may also be a protein obtained, without using the above-mentioned microbial strain, by producing a gene encoding the amino acid sequence by chemical synthesis or the like on the basis of the amino acid sequence of the FEN protein possessed by the above-mentioned microbial strain, causing the gene to be expressed in a heterologous protein expression system (recombinant protein expression system), and isolating the resulting protein by extraction or purification. When the FEN protein is expressed in a heterologous protein expression system, a gene that gives an amino acid sequence modified so as to have the sequence identity within the above-mentioned range, may be used. Incidentally, the information such as the amino acid sequence of the FEN protein possessed by the above-mentioned microbial strain is available from sequence databases (for example, GenBank and NCBI).

The first flap cleaving step is a step of cleaving a first flap of a first cleavage structure formed by a target nucleic acid, a first nucleic acid, and a second nucleic acid.

The first nucleic acid and the second nucleic acid are designed to be hybridizable to a first region of the target nucleic acid and a second region adjacent to the 3' side of the first region, respectively. At this time, the first nucleic acid is designed such that a partial sequence on the 3' side hybridizes to the first region of the target nucleic acid, and the remaining sequence on the 5' side protrudes at one strand and forms a flap. Furthermore, the sequence of the flap is designed so as to hybridize to a second region of a fourth nucleic acid. The second nucleic acid is designed so as to hybridize to the second region of the target nucleic acid. The first region and the second region of the target nucleic acid are designed so as to have nucleotide sequences capable of specifically detecting the target nucleic acid. For example, when applied to SNP analysis, it is preferable to design the first flap such that an SNP site comes to the binding portion of the first region and the second region, that is, the site where three nucleic acids overlap.

The first cleavage structure is a structure in which a partial sequence on the 3' side of the first nucleic acid hybridizes to the first region of the target nucleic acid, the second nucleic acid hybridizes to the second region adjacent to the 3' side of the first region of the target nucleic acid, and the remaining sequence on the 5' side of the first nucleic acid protrudes at one strand and forms a first flap. In the first flap cleaving step, the FEN recognizes this first cleavage structure, cleaves the root of the first flap (phosphodiester bond on the 3' side of the site where three nucleic acids overlap), and releases the first flap (nucleic acid).

It is preferable that the first flap cleaving step is carried out under the conditions in which the pH of the reaction liquid is 7.5 or higher and 9.0 or lower. When the pH is within this range, the effect of enhancing the S/N ratio becomes more remarkable. In addition to the satisfactory S/N ratio, from the viewpoint that detection is enabled in a shorter period of time, the pH of the reaction liquid is preferably in the range of 8.0 or higher and 9.0 or lower, more preferably in the range of 8.2 or higher and 8.8 or lower, and even more preferably in the range of 8.4 or higher and 8.6 or lower.

The first flap cleaving step is preferably carried out under the conditions in which the temperature of the reaction liquid is 55°C or higher and 75°C or lower and is more preferably carried out under the conditions of 55°C or higher and 70°C or lower. When the temperature is within this range, the effect of enhancing the S/N ratio becomes more remarkable. In addition to the satisfactory S/N ratio, from the viewpoint that detection is enabled in a shorter period of time even when the concentration of the target nucleic acid is low, the temperature of the reaction liquid may be, for example, 60°C or higher and may be 65°C or lower. When the temperature of the reaction liquid is 60°C or higher and 65°C or lower, the effect that detection is enabled in a shorter period of time even when the concentration of the target nucleic acid is low, is provided more notably.

It is preferable that the first flap cleaving step is carried out in an aqueous medium including $Mg^{2+}$. From the viewpoint that the effect of enhancing the S/N ratio becomes more remarkable, the concentration of $Mg^{2+}$ in the reaction liquid (aqueous medium) is preferably in the range of 1 mM or greater and 20 mM or less, and more preferably in the range of 2.5 mM or greater and 20 mM or less. In addition to the satisfactory S/N ratio, from the viewpoint that detection is enabled in a shorter period of time, it is even more preferable that the concentration of $Mg^{2+}$ in the reaction liquid (aqueous medium) is in the range of 5 mM or greater and 10 mM or less. The concentration of $Mg^{2+}$ in the reaction liquid (aqueous medium) can be adjusted by, for example, adding a magnesium salt such as magnesium chloride to the reaction liquid.

It is preferable that the first flap cleaving step is carried out in an aqueous medium having a concentration of $K^+$ of 0 mM or greater and 100 mM or less. As a result, the effect of enhancing the S/N ratio becomes more remarkable. Incidentally, when the concentration of $K^+$ is in the above-described range, since there is no large difference in the S/N ratio and the rapidness of the rise even when the concentration of $K^+$ in the reaction liquid (aqueous medium) is 0 mM, it is not always necessary to add $K^+$, and from the viewpoint that the reaction liquid can be simplified, it is more preferable that the concentration of $K^+$ is 0 mM. The concentration of $K^+$ in the reaction liquid (aqueous medium) can be adjusted by, for example, adding a potassium salt such as potassium chloride to the reaction liquid.

The first flap cleaving step may be carried out in any buffer solution. As the buffer solution, for example, a Tris buffer solution, a Bis-Tris buffer solution, and a MOPS buffer solution can be used. From the viewpoint that the effect of enhancing the S/N ratio becomes more remarkable, it is preferable that the first flap cleaving step is carried out in an aqueous solvent having a concentration of trishydroxymethylaminomethane of greater than 0 mM and 300 mM or less. In addition to the effect of enhancing the S/N ratio becoming more remarkable, from the viewpoint that detection is enabled in a shorter period of time, it is more preferable that the concentration of trishydroxymethylaminomethane in the reaction liquid (aqueous medium) is in the range of 5 mM or greater and 300 mM or less.

In the first flap cleaving step, the concentration of the flap endonuclease in the reaction liquid (aqueous medium) may be 0.5 μM or greater and 8 μM or less. As a result, a sufficient effect of enhancing the S/N ratio can be achieved.

It is preferable that the first flap cleaving step is carried out such that the volume of the reaction liquid (aqueous solvent) is 10 aL or more and 10 μL or less. As a result, the effect of enhancing the S/N ratio becomes more remarkable.

The concentrations of the first nucleic acid and the second nucleic acid in the reaction liquid may be each appropriately set to be, for example, in the range of 1 nM or greater and 10 μM or less.

The first flap cleaved in the first flap cleaving step forms a second cleavage structure with a third nucleic acid and a fourth nucleic acid.

The second flap cleaving step is a step of cleaving a second flap of the second cleavage structure formed by a third nucleic acid, the cleaved first flap, and a fourth nucleic acid.

The third nucleic acid is designed so as to be hybridizable to a first region of the fourth nucleic acid. At this time, the third nucleic acid is designed such that a partial sequence on the 3' side hybridizes to the first region of the fourth nucleic acid, and the remaining sequence on the 5' side protrudes at one strand and forms a flap. Furthermore, the first flap is designed so as to hybridize to a second region adjacent to the 3' side of the first region of the fourth nucleic acid.

The second cleavage structure is a structure in which a partial sequence on the 3' side of the third nucleic acid hybridizes to the first region of the fourth nucleic acid, the first flap hybridizes to a second region adjacent to the 3' side of the first region of the fourth nucleic acid, and the remaining portion on the 5' side of the third nucleic acid protrudes at one strand and forms a second flap. In the second flap cleaving step, the FEN recognizes this second cleavage structure, cleaves the root of the second flap (phosphodiester bond on the 3' side of the site where three nucleic acids overlap), and releases the second flap (nucleic acid).

The third nucleic acid and the fourth nucleic acid may be each configured as separate nucleic acid molecules or may be linked by a linker molecule. Examples of the linker molecule include a nucleic acid (that is, the third nucleic acid and the fourth nucleic acid are configured as a single molecular nucleic acid by being bonded by means of a nucleic acid linker) and a spacer composed of an aliphatic compound.

The third nucleic acid may be labeled depending on the detection method used in the detection step. Examples of the label include a fluorescent dye, a radioactive nuclide, a luminescent substance, a phosphorescent substance, and a redox molecule. The labeling method may follow a conventional method in the present technical field. When a fluorescent dye is used as a label, for example, the fluorescent dye may be bonded to the site corresponding to a flap of the third nucleic acid, and a quenching molecule according to the type of the fluorescent dye may be bonded to the site corresponding to a partial sequence on the 3' side of the third nucleic acid. As a result, in the state of the second cleavage structure, fluorescence from the fluorescent dye is not detected by FRET, and fluorescence from the fluorescent dye is detected when the second flap is released, so that detection can be achieved more simply.

Preferred reaction conditions for the second flap cleaving step may be conditions similar to the reaction conditions described in the first flap cleaving step.

The concentrations of the third nucleic acid and the fourth nucleic acid in the reaction liquid may be each appropriately set to be, for example, in the range of 1 nM or greater and 10 $\mu$M or less.

The first flap cleaving step and the second flap cleaving step may be carried out in different reaction vessels or may be carried out in the same reaction vessel. From the viewpoint that the operation becomes simpler, it is preferable that the first flap cleaving step and the second flap cleaving step are carried out in the same reaction vessel. In this case, it is desirable to have the third nucleic acid and the fourth nucleic acid incorporated in advance in the reaction liquid.

The detection step is a step of detecting the presence of the target nucleic acid by detecting the cleaved second flap. The detection step can be carried out by, for example, a method of detecting a band having a size corresponding to the second flap by electrophoresis, and in a case where the second flap has a label, a method of detecting the second flap by fluorescence detection, radioactivity detection, or the like according to the label.

The method for detecting a target nucleic acid according to the present embodiment can be suitably applied to digital measurement due to the enhanced S/N ratio.

A kit according to the present embodiment is a kit for use in the above-mentioned detection method according to the present invention and includes the above-described third nucleic acid, the above-described fourth nucleic acid, the above-described flap endonuclease, and a manual indicating the guidelines for designing the nucleotide sequences of the first nucleic acid and the second nucleic acid according to the nucleotide sequence of the target nucleic acid. Specific embodiments of the first nucleic acid, second nucleic acid, third nucleic acid, and fourth nucleic acid are as described above.

The present invention also relates to an expression vector that has: a nucleic acid sequence encoding an amino acid sequence having a sequence identity of 96% or higher with the amino acid sequence of a flap endonuclease of a microbe selected from the group consisting of Thermococcus kodakarensis strain KOD1, Pyrococcus abyssi strain GE5, and Methanothermobacter thermautotrophicus strain Delta H; and one or a plurality of regulatory sequences operably linked to the nucleic acid sequence.

According to the expression vector according to the present embodiment, an FEN protein that can be suitably used for the above-mentioned detection method according to the present invention can be produced.

A regulatory sequence is a sequence that controls the expression of the FEN protein in a host (for example, a promoter, an enhancer, a ribosome binding sequence, or a transcriptional termination sequence) and can be appropriately selected according to the type of the host. The type of the expression vector can be appropriately selected according to the type of the host from a plasmid vector, a virus vector, a cosmid vector, a phosmid vector, an artificial chromosome vector, and the like.

**Examples**

Hereinafter, the present invention will be more specifically described based on Test Examples. However, the present invention is not intended to be limited to the following Test Examples.

[Test Example 1: Preparation of FEN protein]

Genes encoding various kinds of flap endonuclease (FEN) proteins shown in Table 1 were acquired by a PCR method or chemical synthesis, and the acquired genes were each recombined into an expression vector for Escherichia coli (pET21a). In the case of genes acquired by chemical synthesis, the genes were synthesized so as to provide six histidine residues to the C-terminus. Incidentally, the FEN protein of Test No. 1 (abbreviated name Afu) is an enzyme that has been conventionally used for the ICA method (see Japanese Unexamined Patent Publication No. 2001-518805).

[Table 1]

| Test No. | Accession Number | Species | Abbreviated name |
|---|---|---|---|
| 1 | AF_RS01330 | Archaeoglobus fulgidus | Afu |
| 2 | APE_0115.1 | Aeropyrum pernix K1 | Ape |
| 3 | MTH_RS07825 | Methanothermobacter thermautotrophicus str. Delta H | Mth |
| 4 | PAB_RS03945 | Pyrococcus abyssi GE5 | Pab |
| 5 | TA_RS05360 | Thermoplasma acidophilum DSM1728 | Tac |
| 6 | TK_RS06335 | Thermococcus kodakarensis KOD1 | Tko |
| 7 | SSO_RS00880 | Sulfolobus solfataricus P2 | Sso |
| 8 | STK_RS01185 | Sulfolobus tokodaii str.7 | Sto |
| 9 | WP_014025658.1 | Pyrolobus fumarii | Pfuma |
| 10 | WP_055410231.1 | Pyrodictium delaneyi | Pdela |
| 11 | WP_011762435.1 | Pyrobaculum islandicum | Pisla |
| 12 | AET33596.1 | Pyrobaculum ferrireducens | Pferr |
| 13 | AFA40545.1 | Pyrobaculum oguniense | Pogun |
| 14 | WP_013142419.1 | Staphylothermus hellenicus | Shell |
| 15 | AFZ71119.1 | Caldisphaera lagunensis | Clagu |
| 16 | WP_010870964.1 | Methanocaldococcus j annaschii | Mjann |
| 17 | WP_015732726.1 | Methanocaldococcus vulcanius | Mvulc |
| 18 | WP_013798814.1 | Methanotorris igneus | Migne |
| 19 | WP_048092128.1 | Geoglobus acetivorans | Gacet |
| 20 | WP_048094195.1 | Geoglobus ahangari | Gahan |
| 21 | WP_012940856.1 | Archaeoglobus profundus | Aprof |
| 22 | WP_074358343.1 | Methanothermobacter wolfeii | Mwolf |
| 23 | WP_013295054.1 | Methanothermobacter marburgensis | Mmarb |
| 24 | WP_018154436.1 | Methanothermococcus thermolithotrophicus | Mthermo |
| 25 | PF_RS07095 | Pyrococcus furiosus DSM3638 | Pfu |
| 26 | WP_010885498.1 | Pyrococcus horikoshii | Phori |
| 27 | WP_088884722.1 | Thermococcus gorgonarius | Tgorg |
| 28 | WP_015858814.1 | Thermocuccus gammatolerans | Tgamm |
| 29 | WP_010477429.1 | Thermococcus zilligii | Tzill |

Escherichia coli (BL21-CodonPlus (DE3)) was transformed with the established expression vector. Escherichia coli cells after transformation were seeded in LB medium including 100 μg/mL ampicillin and 35 μg/mL chloramphenicol and were cultured at 37°C until the $OD_{600}$ value reached about 0.5. Next, 1 M IPTG was added thereto until the final concentration reached 0.5 mM, and the cells were cultured at 25°C for 16 hours to express a large amount of FEN protein.

The cultured Escherichia coli (medium 1 L) cells were collected by centrifugal separation and were resuspended in a buffer solution (25 mL). The resuspended Escherichia coli cells were crushed by ultrasonication and then centrifuged to collect the supernatant. Next, the collected supernatant was heated at 80°C for 20 minutes and then centrifuged to collect the supernatant. Next, polyethyleneimine was added to the collected supernatant, and then the mixture was centrifuged to collect the supernatant. Ammonium sulfate was added to the collected supernatant up to 80% saturation, and a precipitate thus formed was collected. The obtained precipitate was resuspended in a buffer solution including ammonium sulfate, insoluble materials were removed, subsequently the solution was fractioned by hydrophobic interaction chromatography, and a fraction including an intended FEN protein was collected. The collected fraction was dialyzed against a buffer solution, and then the FEN protein was further purified by heparin affinity chromatography. In the case of a protein having a histidine residue at the C-terminus, the cultured Escherichia coli cells (medium 1 L) were collected by centrifugal separation and were resuspended in a buffer solution (25 mL). The resuspended Escherichia coli cells were crushed by ultrasonication and then were centrifuged to collect the supernatant. Next, the collected supernatant was heated at 60°C to 80°C for 20 minutes and then centrifuged to collect the supernatant. Next, the collected supernatant was added to TALON metal affinity resin (Clontech Laboratories, Inc.), the mixture was fractioned by immobilized metal affinity chromatography by using a buffer solution including imidazole, and a fraction including an intended FEN protein was collected.

Incidentally, the FEN proteins of Test No. 7 (abbreviated name Sso) and Test No. 8 (abbreviated name Sto) were aggregated and precipitated during the above-described purification process and could not be purified, and therefore, the FEN proteins were excluded from the subsequent studies.

For each of the purified FEN proteins, ultraviolet absorption was measured, and the concentration was calculated from the molar extinction coefficient at a wavelength of 280 nm. Furthermore, each of the purified FEN proteins was analyzed by SDS-PAGE, and a single band of expected size was confirmed. Furthermore, some minor bands were also detected; however, it was confirmed that there was no non-specific nuclease activity.

[Test Example 2: Evaluation of flap cleaving activity]

The flap cleaving activity of the FEN proteins purified in Test Example 1 was evaluated. A cleavage structure was formed by a target nucleic acid (5'-GGTGATCGTTCGCTACATGTCGTCAGGATTCCAGGCAG-3': SEQ ID NO:1), a first nucleic acid (5'-FITC-AGACACATGGTATGTAGCGAACGATCACC-3': SEQ ID NO:2), and a second nucleic acid (5'-CTGCCTGGAATCCTGACGAC-3': SEQ ID NO:3). In this cleavage structure, a partial sequence (eleven bases from the 5'-end to the underlined T) on the 5' side of the first nucleic acid protrudes at one strand as a flap.

The cleaving reaction was carried out by preparing a reaction solution at the composition of 50 mM Tris-HCl (pH 7.6), 2.5 mM $MgCl_2$, 10 nM substrate DNA (equimolar mixture of the above-described target nucleic acid, first nucleic acid, and second nucleic acid), and 0.5 μM FEN protein, and incubating this reaction solution at 55°C for 10 minutes.

After completion of the reaction, electrophoresis was performed using 12% polyacrylamide gel including 8 M urea. Next, the fluorescence intensities of the uncleaved cleavage structure (band at the position of 38 bases) and the cleaved flap (band at the position of 11 bases) were measured by using an image analyzer (Typhoon Trio+, manufactured by GE Healthcare Systems). From the measured fluorescence intensities, the flap cleaving activity was calculated by the following formula. The results are shown in Table 2.

$$\text{Flap cleaving activity} = (\text{Fluorescence intensity of band at position of 11 bases}) / \{(\text{fluorescence intensity of band at position of 11 bases}) + (\text{fluorescence intensity of band at position of 38 bases})\} \times 100 \, (\%)$$

[Table 2]

| Test No. | Origin of FEN protein | Flap cleaving activity (%) |
|---|---|---|
| 1 | Afu | 94 |

(continued)

| Test No. | Origin of FEN protein | Flap cleaving activity (%) |
|---|---|---|
| 2 | Ape | 94 |
| 3 | Mth | 97 |
| 4 | Pab | 97 |
| 5 | Tac | 96 |
| 6 | Tko | 96 |
| 7 | Sso | - |
| 8 | Sto | - |
| 9 | Pfuma | 90 |
| 10 | Pdela | 93 |
| 11 | Pisla | 83 |
| 12 | Pferr | 84 |
| 13 | Pogun | 11 |
| 14 | Shell | 95 |
| 15 | Clagu | 54 |
| 16 | Mjann | 90 |
| 17 | Mvulc | 94 |
| 18 | Migne | 94 |
| 19 | Gacet | 82 |
| 20 | Gahan | 83 |
| 21 | Aprof | 89 |
| 22 | Mwolf | 93 |
| 23 | Mmarb | 91 |
| 24 | Mthermo | 97 |
| 25 | Pfu | 96 |
| 26 | Phori | 94 |
| 27 | Tgorg | 88 |
| 28 | Tgamm | 92 |
| 29 | Tzill | 84 |

As shown in Table 2, sufficient flap cleaving activity was recognized in the FEN proteins other than Test No. 13 (abbreviated name Pogun) and Test No. 15 (abbreviated name Clagu).

[Test Example 3: Two-stage evaluation of flap cleaving activity]

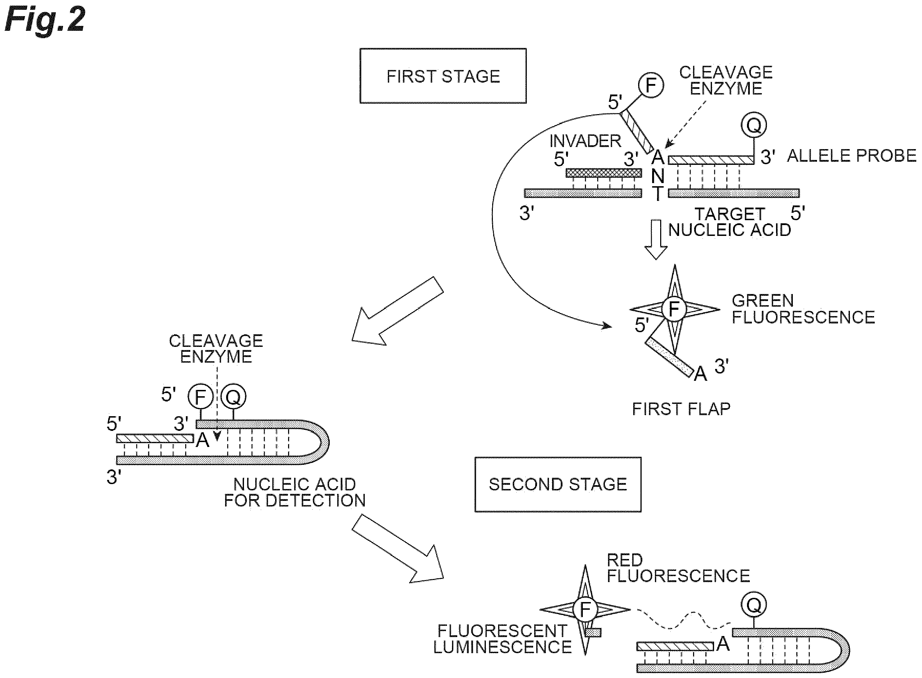

For the FEN proteins whose flap cleaving activity was recognized in Test Example 2, the flap cleaving activity in a two-stage flap cleaving reaction that is conventionally employed in the ICA method was evaluated. An outline of the evaluation system is as shown in FIG. 2. The evaluation system shown in FIG. 2 is equivalent to the reaction system generally used in the ICA method, except that detection of a flap cleaving reaction of the first stage (reaction of cleaving the first flap of the first cleavage structure) by the FEN protein was enabled by fluorescently labeling an allele probe.

In the evaluation system shown in FIG. 2, a first cleavage structure is formed of a target nucleic acid (nucleotide sequence: 5'-GGTGATCGTTCGCTACATGTCGTCAGGATTCCAGGCAG-3': SEQ ID NO:4), allele probes corresponding to a first nucleic acid and a second nucleic acid, respectively (nucleotide sequence: 5'-FAM-AGACACATGGTATGTAGCGAACGATCACC-BHQ1-3': SEQ ID NO:5), and an invader oligonucleotide (nucleotide sequence: 5'-CTGCCTGGAATCCTGACGAC-3': SEQ ID NO:6). At the 5' end and the 3' end of the allele probe, a fluorescent molecule (FAM) and a quenching molecule (BHQ1: a thymine residue modified with Black Hole Quencher) are respectively bonded, and when the first flap is cleaved by the FEN protein (first stage flap cleaving reaction), green fluorescence of FAM is detected. The cleaved first flap forms a second cleavage structure with a nucleic acid for detection (nucleotide sequence: 5'-RedmondRed-TCT-EclipseQuencher-TCGGCCTTTTGGCCGAGAGACTCCGCGTCCGT-3': SEQ ID NO:7) corresponding to a third nucleic acid and a fourth nucleic acid bonded by a linker molecule (hereinafter, a portion of the nucleotide sequence of the nucleic acid for detection). A fluorescent molecule (RedmondRED) is bonded to the 5' end of the nucleic acid for detection, and a quenching molecule (Eclipse Quencher) is inserted between the third residue and the fourth residue from the 5' terminus of the nucleic acid for detection, so that when the second flap is cleaved by the FEN protein (second-stage flap cleaving reaction), red fluorescence of RedmondRED is detected.

For the FEN proteins of Test No. 1 (abbreviated name Afu), Test No. 3 (abbreviated name Mth), Test No. 4 (abbreviated name Pab), Test No. 6 (abbreviated name Tko), and Test No. 25 (abbreviated name Pfu), the two-stage flap cleaving reaction was evaluated. The two-stage flap cleaving reaction was carried out by preparing a reaction solution at the composition of 1 μM allele probe (first nucleic acid), 1 μM invader oligonucleotide (second nucleic acid), 4 μM nucleic acid for detection (third nucleic acid and fourth nucleic acid), 50 mM Tris-HCl (pH 8.5), 0.05 v/v% Tween20, 20 mM MgCl$_2$, 0.086 mg/mL or 0.1 mg/mL FEN protein, and 0 M, 1.5 pM, 100 nM, or 1 μM target nucleic acid, and incubating the reaction solution at 65°C for 60 minutes while measuring the fluorescence intensity in real-time by using LightCycler480 (registered trademark) (manufactured by Roche Diagnostics International AG).

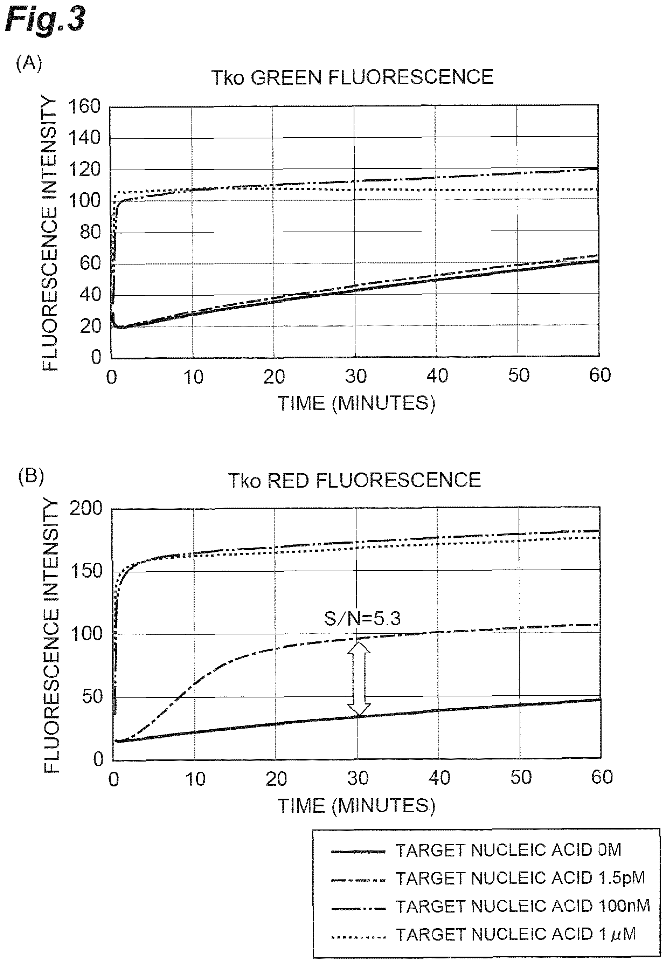

An example of the results of real-time measurement of the fluorescence intensity is shown in FIG. 3. FIG. 3 shows the measurement results at the time of using a FEN protein of Test No. 6 (abbreviated name Tko). FIG. 3(A) is a graph showing the measurement results for green fluorescence (fluorescence from the released first flap). FIG. 3(B) is a graph showing the measurement results for red fluorescence (fluorescence from the released second flap).

As shown in FIG. 3, it can be confirmed that both green fluorescence and red fluorescence tend to increase according to the concentration of the target nucleic acid. Incidentally, when the concentration of the target nucleic acid is 100 nM and 1 μM, since the target nucleic acid concentration is high, it is considered that the fluorescence intensity is saturated. Furthermore, when the concentration of the target nucleic acid is 1.5 pM, the difference between red fluorescence and the background (when the concentration of the target nucleic acid is 0 M) is larger compared with the difference of green fluorescence; however, this is because an amplification of the signal (released second flap) has occurred as the released first flap repeatedly forms the second cleavage structure with the nucleic acid for detection.

The S/N ratio was calculated from the ratio between the red fluorescence intensity (signal) in the case where the concentration of the target nucleic acid was 1.5 pM at the time point after 30 minutes from the initiation of reaction and the red fluorescence intensity (noise) in the case where the concentration of the target nucleic acid was 0 M. The results are shown in Table 3.

[Table 3]

| Test No. | Origin of FEN protein | S/N ratio |
|----------|----------------------|-----------|
| 1 | Afu | 3.2 |
| 3 | Mth | 3.3 |
| 4 | Pab | 4.8 |
| 6 | Tko | 5.3 |
| 25 | Pfu | 2.4 |

As shown in Table 3, the FEN proteins of Test No. 3 (abbreviated name Mth), Test No. 4 (abbreviated name Pab), and Test No. 6 (abbreviated name Tko) showed higher S/N ratios as compared with the FEN protein of Test No. 1 (abbreviated name Afu), which has been conventionally used. On the other hand, the FEN protein of Test No. 25 (abbreviated name Pfu) had a lower S/N ratio than the FEN protein of Test No. 1 (abbreviated name Afu), which has been conventionally used.

For the FEN proteins other than those described above and the FEN protein of Test No. 6 (abbreviated name Tko), the two-stage flap cleaving reaction was evaluated under the following conditions. The two-stage flap cleaving reaction was carried out by preparing a reaction solution at the composition of 1 μM allele probe (first nucleic acid), 1 μM invader oligonucleotide (second nucleic acid), 2 μM nucleic acid for detection (third nucleic acid and fourth nucleic acid), 50 mM Tris-HCl (pH 8.5), 0.05 v/v% Tween20, 2.5 mM MgCl$_2$, 1.16 μM (Test No. 10 only) or 7.74 μM FEN protein, and 0 M, 1.5 pM, 30 pM, or 100 pM target nucleic acid, and incubating the reaction solution at 65°C for 60 minutes while measuring the fluorescence intensity in real-time by using LightCycler480 (registered trademark) (manufactured by Roche Diagnostics International AG).

The S/N ratio was calculated from the ratio between the red fluorescence intensity (signal) in the case where the concentration of the target nucleic acid was 1.5 pM at the time point after 30 minutes from the initiation of reaction and the red fluorescence intensity (noise) in the case where the concentration of the target nucleic acid was 0 M. The results are shown in Table 4.

[Table 4]

| Test No. | Origin of FEN protein | S/N ratio | Enzyme concentration ($\mu$M) |
|---|---|---|---|
| 6 | Tko | 2.57 | 7.74 |
| | | 4.13 | 1.16 |
| 9 | Pfuma | 1.12 | 7.74 |
| 10 | Pdela | 1.07 | 1.16 |
| 11 | Pisla | 1.25 | 7.74 |
| 12 | Pferr | 1.16 | 7.74 |
| 13 | Pogun | 1.00 | 7.74 |
| 14 | Shell | 1.30 | 7.74 |
| 15 | Clagu | 1.00 | 7.74 |
| 16 | Mjann | 1.12 | 7.74 |
| 17 | Mvulc | 0.96 | 7.74 |
| 18 | Migne | 1.00 | 7.74 |
| 19 | Gacet | 1.38 | 7.74 |
| 20 | Gahan | 1.20 | 7.74 |
| 21 | Aprof | 1.16 | 7.74 |
| 22 | Mwolf | 1.25 | 1.16 |
| 23 | Mmarb | 1.11 | 1.16 |
| 24 | Mthermo | 1.00 | 1.16 |
| 26 | Phori | 1.42 | 1.16 |
| 27 | Tgorg | 0.82 | 1.16 |
| 28 | Tgamm | 1.00 | 1.16 |
| 29 | Tzill | 1.24 | 1.16 |

Regarding the FEN proteins of Test No. 9 to Test No. 24 and Test No. 26 to Test No. 29, none of them showed a high S/N ratio.

[Test Example 4: Examination of reaction conditions for flap cleaving reaction]

Examination of the reaction conditions for the flap cleaving reaction was conducted by using the FEN proteins of Test No. 3 (abbreviated name Mth) and Test No. 6 (abbreviated name Tko), with an evaluation system similar to that of Test Example 3.

<pH>

The reaction liquid composition was set to a composition including 50 mM Tris-HCl having the pH adjusted to pH 7.5, pH 8.0, pH 8.5, or pH 9.0 as a base; 1 $\mu$M allele probe (first nucleic acid); 1 $\mu$M invader oligonucleotide (second nucleic acid); 2 $\mu$M nucleic acid for detection (third nucleic acid and fourth nucleic acid); 0.05 v/v% Tween20; 2.5 mM MgCl$_2$; 0.5 $\mu$M FEN protein; and 0 M, 100 pM, or 1 nM target nucleic acid. The amount of the reaction liquid was set to 10 µL. A flap cleaving reaction was carried out by preparing the reaction solution and then incubating the reaction solution at 67°C for 60 minutes while measuring the fluorescence intensity in real-time by using LightCycler480 (registered trademark) (manufactured by Roche Diagnostics AG). Incidentally, for the examination of pH, only the FEN protein of Test No. 6 (abbreviated name Tko) was used.

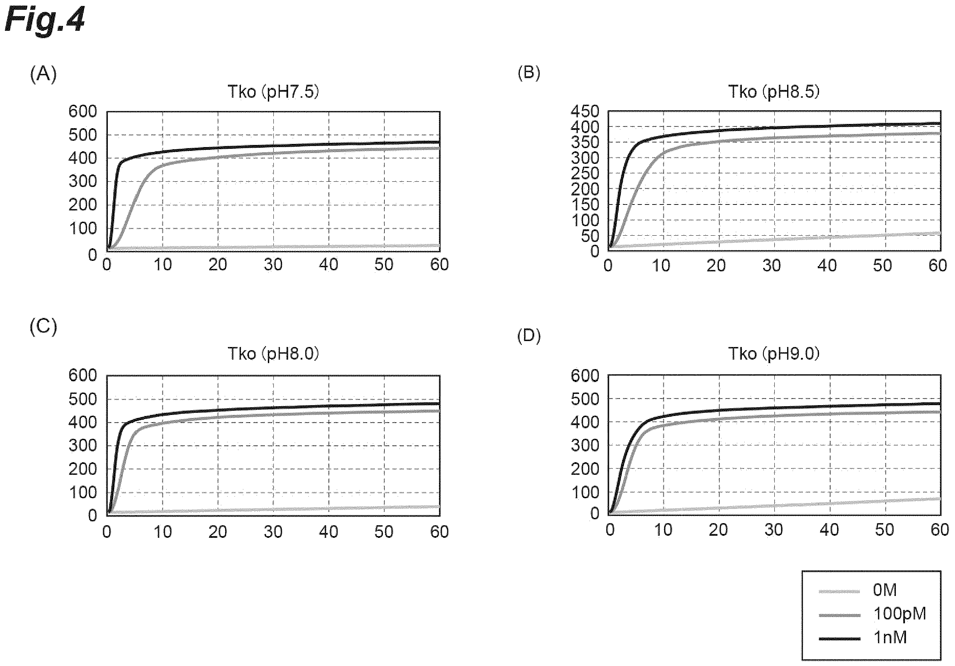

The S/N ratio was calculated from the ratio between the red fluorescence intensity (signal) in the case where the concentration of the target nucleic acid was 100 pM at the time point after 30 minutes from the initiation of reaction and the red fluorescence intensity (noise) in the case where the concentration of the target nucleic acid was 0 M. The results are shown in Table 5. Furthermore, FIG. 4 shows the results (graph) for the real-time measurement of fluorescence intensity. The axis of ordinate of the graph represents the fluorescence intensity, the axis of abscissa represents the time (minutes) from the initiation of reaction, and FIGS. 4(A) to 4(D) show the results of measuring the fluorescence intensity at pH 7.5, pH 8.0, pH 8.5, or pH 9.0 using the FEN protein of Test No. 6 (abbreviated name Tko).

[Table 5]

| Test No. | Origin of FEN protein | pH | S/N ratio |
|---|---|---|---|
| 6 | Tko | 7.5 | 21.10 |
| | | 8.0 | 15.54 |
| | | 8.5 | 9.84 |
| | | 9.0 | 10.37 |

As shown in FIG. 4 and Table 5, a satisfactory S/N ratio was recognized when the pH of the reaction liquid was in the range of 7.5 or higher and 9.0 or lower. Furthermore, in addition to a satisfactory S/N ratio, an effect that the rise of the red fluorescence intensity occurred more rapidly was recognized near pH 8.5. As the rise of the red fluorescence intensity is more rapid, detection is enabled in a shorter period of time.

<Temperature>

The reaction liquid composition was adjusted to 1 µM allele probe (first nucleic acid); 1 µM invader oligonucleotide (second nucleic acid); 2 µM nucleic acid for detection (third nucleic acid and fourth nucleic acid); 50 mM Tris-HCl (pH 8.5); 0.05 v/v% Tween20; 2.5 mM $MgCl_2$; 0.5 µM FEN protein; and 0 M, 30 pM, or 1 nM target nucleic acid. The amount of the reaction liquid was set to 10 µL. A flap cleaving reaction was carried out by preparing a reaction solution, and then incubating the reaction solution at 55°C, 60°C, 65°C, 67°C, or 70°C for 60 minutes while measuring the fluorescence intensity in real time using LightCycler480 (registered trademark) (manufactured by Roche Diagnostics International AG).

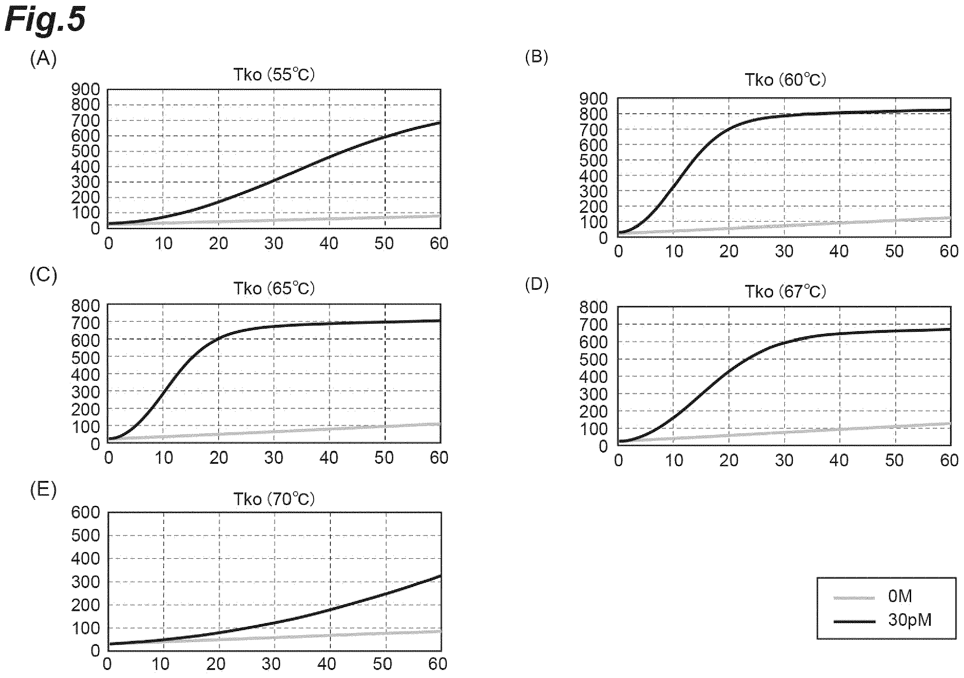

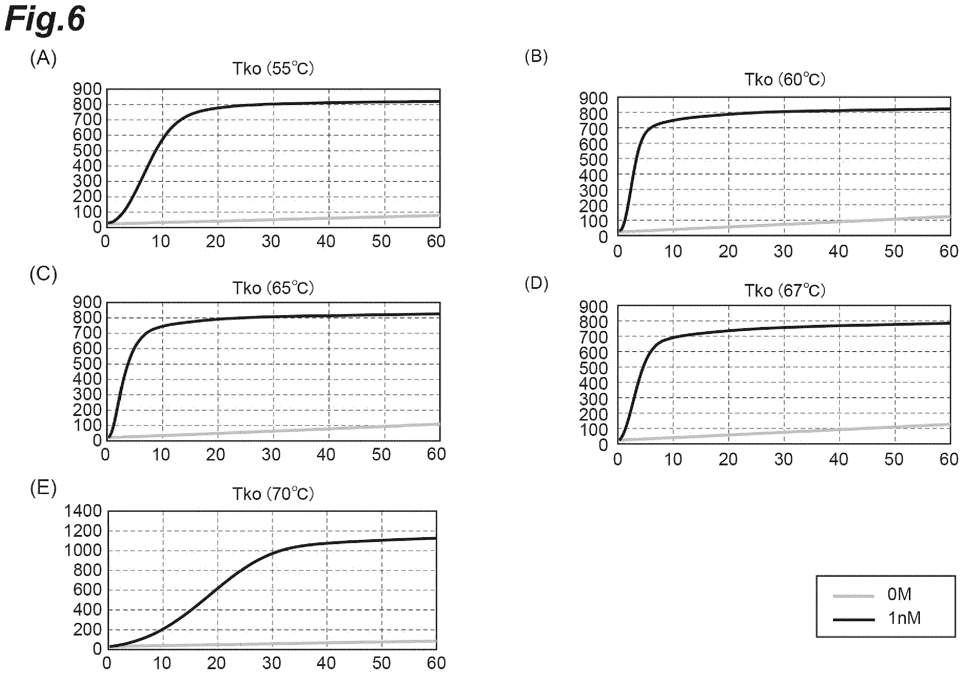

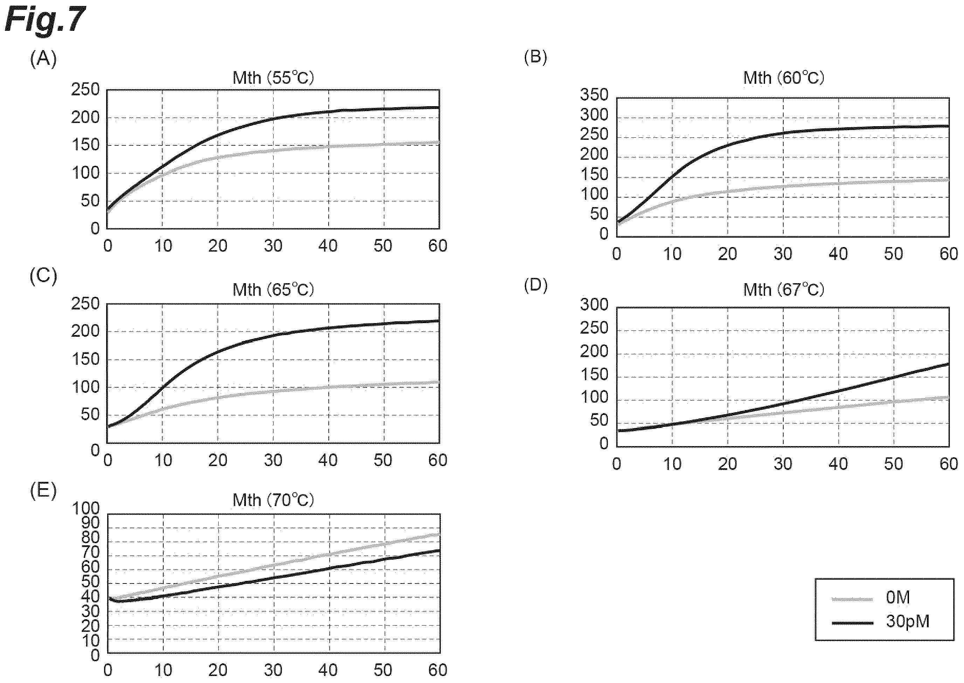

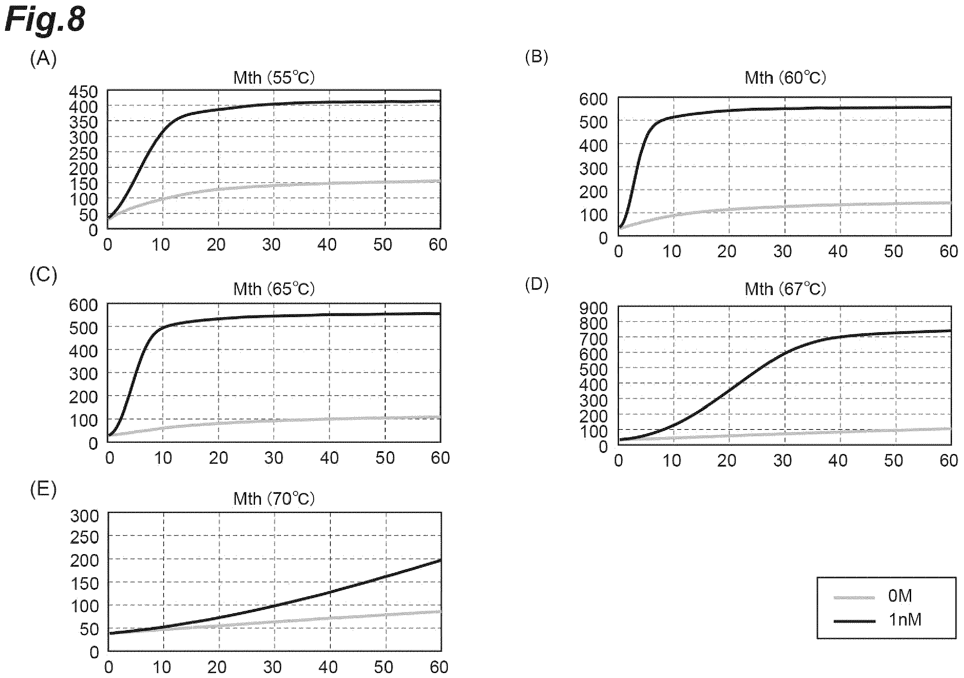

The S/N ratio was calculated from the ratio between the red fluorescence intensity (signal) in the case where the concentration of the target nucleic acid was 30 pM or 1 nM at the time point after 30 minutes from the initiation of reaction and the red fluorescence intensity (noise) in the case where the concentration of the target nucleic acid was 0 M. The results are shown in Table 6. Furthermore, FIG. 5 to FIG. 8 show the results (graph) for the real-time measurement of fluorescence intensity. The axis of ordinate of the graph represents the fluorescence intensity, the axis of abscissa represents the time (minutes) from the initiation of reaction, and FIGS. 5(A) to 5(E) show the results of measuring the fluorescence intensity at a concentration of the target nucleic acid of 30 pM at 55°C, 60°C, 65°C, 67°C, or 70°C using the FEN protein of Test No. 6 (abbreviated name Tko). FIGS. 6(A) to 6(E) show the results of measuring the fluorescence intensity at a concentration of the target nucleic acid of 1 nM at 55°C, 60°C, 65°C, 67°C, or 70°C using the FEN protein of Test No. 6 (abbreviated name Tko). FIGS. 7(A) to 7(E) show the results of measuring the fluorescence intensity at a concentration of the target nucleic acid of 30 pM at 55°C, 60°C, 65°C, 67°C, or 70°C using the FEN protein of Test No. 3 (abbreviated name Mth). FIGS. 8(A) to 8(E) show the results of measuring the fluorescence intensity at a concentration of the target nucleic acid of 1 nM at 55°C, 60°C, 65°C, 67°C, or 70°C using the FEN protein of Test No. 3 (abbreviated name Mth).

[Table 6]

| Test No. | Origin of FEN protein | Target nucleic acid concentration | Temperature (°C) | S/N ratio |
|---|---|---|---|---|
| 3 | Mth | 1 nM | 55 | 2.38 |
| | | | 60 | 4.09 |
| | | | 65 | 5.88 |
| | | | 67 | 3.28 |
| | | | 70 | 2.77 |
| | | 30 pM | 55 | 1.23 |
| | | | 60 | 1.70 |
| | | | 65 | 2.41 |
| | | | 67 | 12.15 |
| | | | 70 | 1.11 |
| 6 | Tko | 1 nM | 55 | 13.98 |
| | | | 60 | 10.30 |
| | | | 65 | 10.37 |
| | | | 67 | 11.68 |
| | | | 70 | 18.24 |
| | | 30 pM | 55 | 3.50 |
| | | | 60 | 8.09 |
| | | | 65 | 8.39 |
| | | | 67 | 9.58 |
| | | | 70 | 3.09 |

As shown in FIGS. 5 to 8 and Table 6, a satisfactory S/N ratio was recognized when the temperature of the reaction liquid was in the range of 55°C or higher and 70°C or lower. Furthermore, with regard to the FEN protein of Test No. 6 (abbreviated name Tko), in addition to a satisfactory S/N ratio, an effect that the rise of the red fluorescence intensity occurred more rapidly even when the concentration of the target nucleic acid was low, was recognized at a temperature in the range of 60°C or higher and 70°C or lower. A similar effect was recognized with the FEN protein of Test No. 3 (abbreviated name Mth) at a temperature in the range of 55°C or higher and 65°C or lower.

<Mg$^{2+}$ ion concentration>

The reaction liquid composition was adjusted to 1 $\mu$M allele probe (first nucleic acid); 1 $\mu$M invader oligonucleotide (second nucleic acid); 2 $\mu$M nucleic acid for detection (third nucleic acid and fourth nucleic acid); 50 mM Tris-HCl (pH 8.5); 0.05 v/v% Tween20; 1 mM, 2.5 mM, 5 mM, 10 mM, or 20 mM MgCl$_2$; 0.5 $\mu$M FEN protein; and 0 M, 100 pM, or 1 nM target nucleic acid. The amount of the reaction liquid was set to 10 $\mu$L. A flap cleaving reaction was carried out by preparing the reaction solution, and then incubating the reaction solution at 67°C for 60 minutes while measuring the fluorescence intensity in real-time using LightCycler480 (registered trademark) (manufactured by Roche Diagnostics AG). Incidentally, for the examination of the Mg$^{2+}$ ion concentration, only the FEN protein of Test No. 6 (abbreviated name Tko) was used.

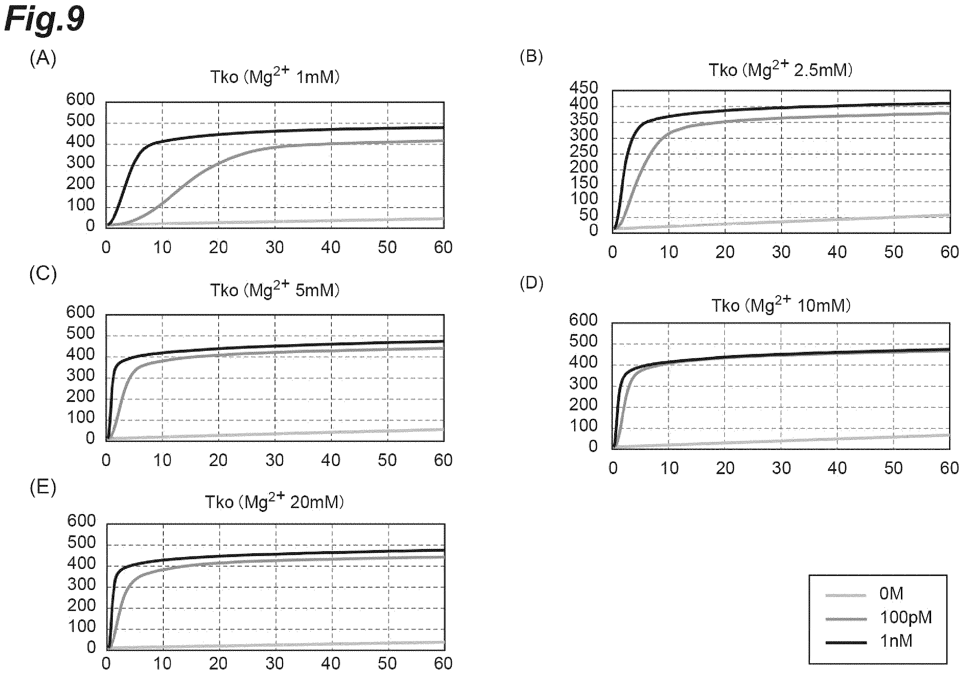

The S/N ratio was calculated from the ratio between the red fluorescence intensity (signal) in the case where the concentration of the target nucleic acid was 100 pM at the time point after 30 minutes from the initiation of reaction and the red fluorescence intensity (noise) in the case where the concentration of the target nucleic acid was 0 M. The results are shown in Table 7. Furthermore, FIG. 9 shows the results (graph) for the real-time measurement of fluorescence intensity. The axis of ordinate of the graph represents the fluorescence intensity, the axis of abscissa represents the time (minutes) from the initiation of reaction, and FIGS. 9(A) to 9(E) show the results of measuring the fluorescence intensity at a Mg$^{2+}$ concentration of 1 mM, 2.5 mM, 5 mM, 10 mM, or 20 mM using the FEN protein of Test No. 6 (abbreviated name Tko).

[Table 7]

| Test No. | Origin of FEN protein | $Mg^{2+}$ concentration (mM) | S/N ratio |
|---|---|---|---|
| 6 | Tko | 1 | 26.29 |
| | | 2.5 | 15.86 |
| | | 5 | 21.37 |
| | | 10 | 16.81 |
| | | 20 | 37.45 |

As shown in FIG. 9 and Table 7, a satisfactory S/N ratio was recognized when the $Mg^{2+}$ concentration in the reaction liquid was in the range of 1 mM or greater and 20 mM or less (preferably in the range of 2.5 mM or greater and 20 mM or less). Furthermore, when the $Mg^{2+}$ concentration was in the range of 5 mM or greater and 10 mM or less, in addition to a satisfactory S/N ratio, an effect that the rise of the red fluorescence intensity occurred more rapidly was recognized.

<$K^+$ ion concentration>

The reaction liquid composition was adjusted to 1 μM allele probe (first nucleic acid); 1 μM invader oligonucleotide (second nucleic acid); 2 μM nucleic acid for detection (third nucleic acid and fourth nucleic acid); 50 mM Tris-HCl (pH 8.5); 0.05 v/v% Tween20; 2.5 mM $MgCl_2$; 0 mM, 25 mM, 50 mM, 100 mM, or 200 mM KCl; 0.5 μM FEN protein; and 0 M, 1 nM, or 10 nM target nucleic acid. The amount of the reaction liquid was set to 10 μL. A flap cleaving reaction was carried out by preparing the reaction solution, and then incubating the reaction solution at 67°C for 60 minutes while measuring the fluorescence intensity in real-time using LightCycler480 (registered trademark) (manufactured by Roche Diagnostics AG).

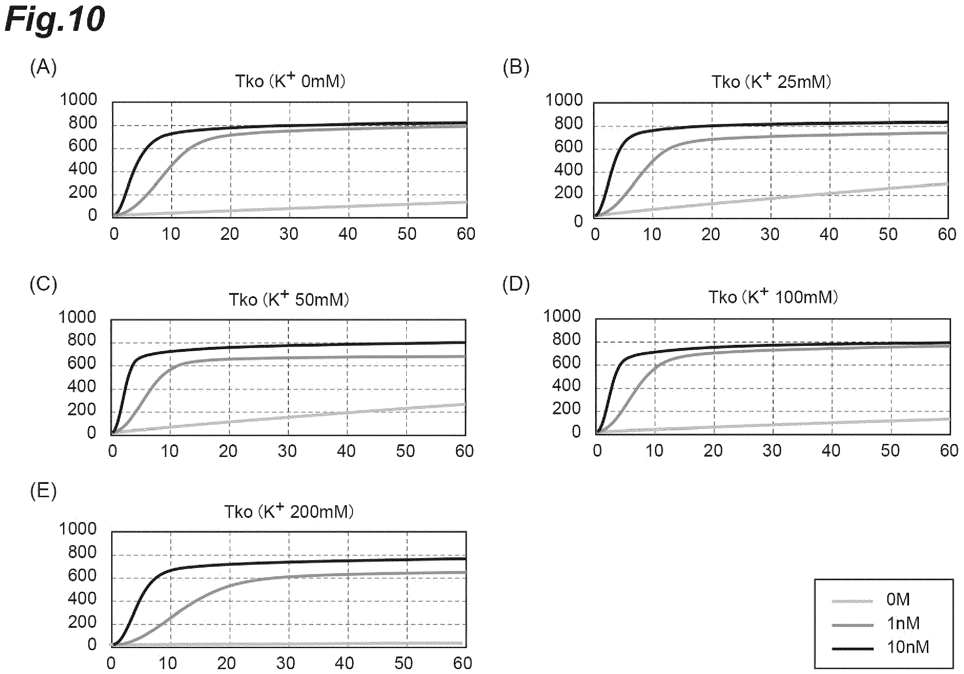

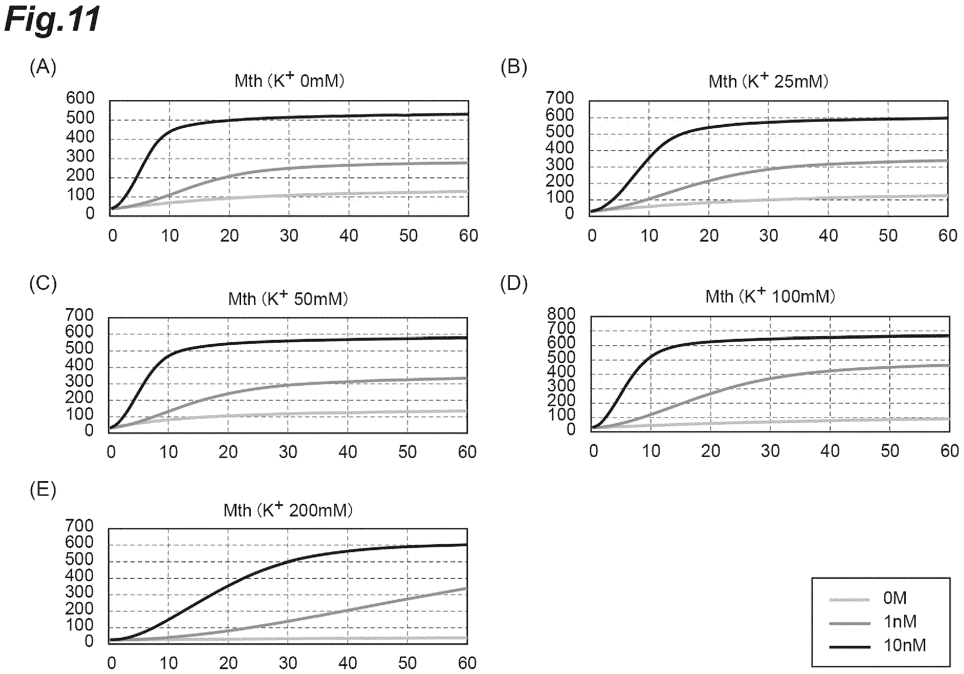

The S/N ratio was calculated from the ratio between the red fluorescence intensity (signal) in the case where the concentration of the target nucleic acid was 1 nM at the time point after 30 minutes from the initiation of reaction and the red fluorescence intensity (noise) in the case where the concentration of the target nucleic acid was 0 M. The results are shown in Table 8. Furthermore, FIGS. 10 and 11 show the results (graph) for the real-time measurement of fluorescence intensity. The axis of ordinate of the graph represents the fluorescence intensity, the axis of abscissa represents the time (minutes) from the initiation of reaction, and FIGS. 10(A) to 10(E) show the results of measuring the fluorescence intensity at a $K^+$ concentration of 0 mM, 25 mM, 50 mM, 100 mM, or 200 mM using the FEN protein of Test No. 6 (abbreviated name Tko). FIGS. 11(A) to 11(E) show the results of measuring the fluorescence intensity at a $K^+$ concentration of 0 mM, 25 mM, 50 mM, 100 mM, or 200 mM using the FEN protein of Test No. 3 (abbreviated name Mth).

[Table 8]

| Test No. | Origin of FEN protein | $K^+$ concentration (mM) | S/N ratio |
|---|---|---|---|
| 3 | Mth | 0 | 2.30 |
| | | 25 | 2.85 |
| | | 50 | 2.48 |
| | | 100 | 5.33 |
| | | 200 | 4.27 |
| 6 | Tko | 0 | 9.18 |
| | | 25 | 4.02 |
| | | 50 | 4.27 |
| | | 100 | 8.78 |
| | | 200 | 19.19 |

As shown in FIGS. 10 and 11 and Table 8, a satisfactory S/N ratio was recognized when the $K^+$ concentration in the reaction liquid was in the range of 100 mM or less. Furthermore, since there was no significant difference in the

14

S/N ratio and the rapidness of the rise within this range, it was found that $K^+$ is not always necessary (0 mM is acceptable).

<Tris (trishydroxymethylaminomethane) concentration>

The reaction liquid composition was adjusted to 1 μM allele probe (first nucleic acid); 1 μM invader oligonucleotide (second nucleic acid); 2 μM nucleic acid for detection (third nucleic acid and fourth nucleic acid); 5 mM, 20 mM, 50 mM, 100 mM, 200 mM, 300 mM, 400 mM, or 500 mM Tris-HCl (pH 8.5); 0.05 v/v% Tween20; 5 mM $MgCl_2$; 0.5 μM FEN protein; and 0 M, 100 pM, or 1 nM target nucleic acid. The amount of the reaction liquid was set to 10 μL. A flap cleaving reaction was carried out by preparing the reaction solution, and then incubating the reaction solution at 67°C for 60 minutes while measuring the fluorescence intensity in real-time using LightCycler480 (registered trademark) (manufactured by Roche Diagnostics AG).

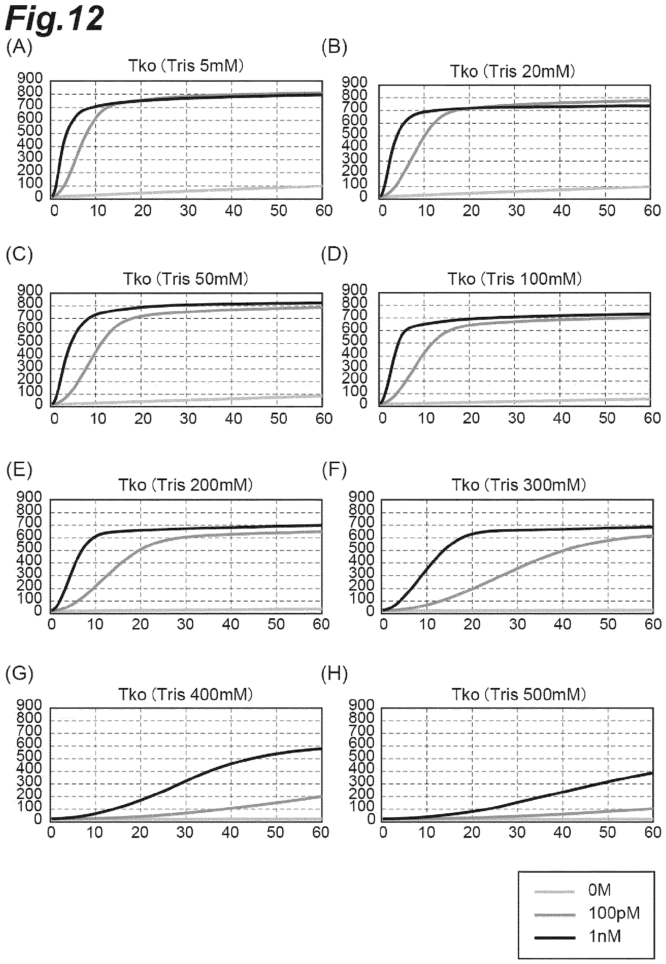

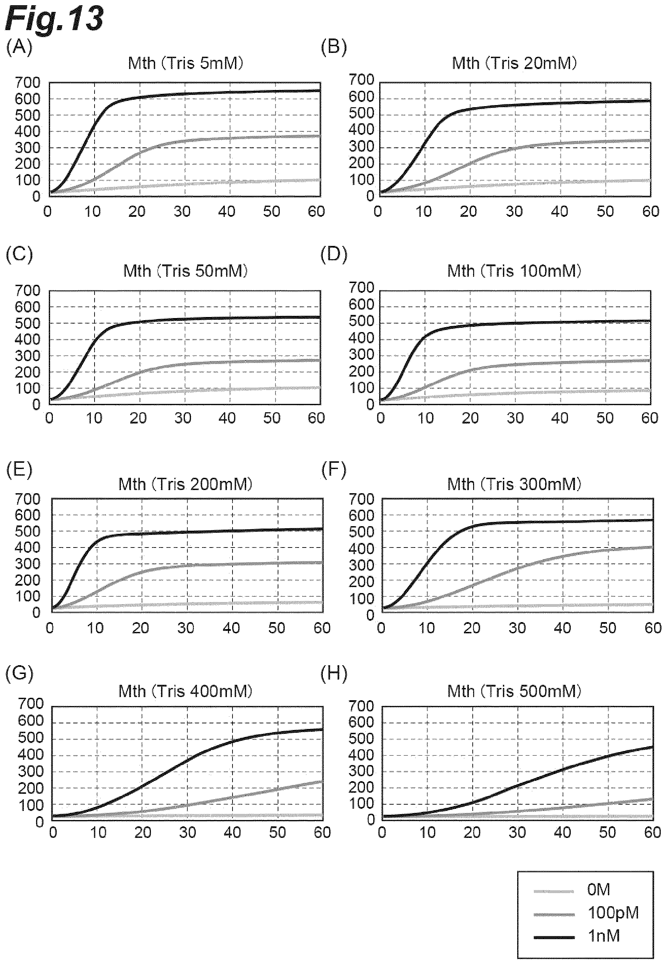

The S/N ratio was calculated from the ratio between the red fluorescence intensity (signal) in the case where the concentration of the target nucleic acid was 100 pM at the time point after 30 minutes from the initiation of reaction and the red fluorescence intensity (noise) in the case where the concentration of the target nucleic acid was 0 M. The results are shown in Table 9. Furthermore, FIGS. 12 and 13 show the results (graph) for the real-time measurement of fluorescence intensity. The axis of ordinate of the graph represents the fluorescence intensity, the axis of abscissa represents the time (minutes) from the initiation of reaction, and FIGS. 12(A) to 12(H) show the results of measuring the fluorescence intensity at a Tris concentration of 5 mM, 20 mM, 50 mM, 100 mM, 200 mM, 300 mM, 400 mM, or 500 mM using the FEN protein of Test No. 6 (abbreviated name Tko). FIGS. 13(A) to 13(H) show the results of measuring the fluorescence intensity at a Tris concentration of 5 mM, 20 mM, 50 mM, 100 mM, 200 mM, 300 mM, 400 mM, or 500 mM using the FEN protein of Test No. 3 (abbreviated name Mth).

[Table 9]

| Test No. | Origin of FEN protein | Tris concentration (mM) | S/N ratio |
|----------|-----------------------|-------------------------|-----------|
| 3 | Mth | 5 | 4.56 |
|   |     | 20 | 4.01 |
|   |     | 50 | 3.01 |
|   |     | 100 | 3.49 |
|   |     | 200 | 5.54 |
|   |     | 300 | 6.85 |
|   |     | 400 | 3.20 |
|   |     | 500 | 2.38 |
| 6 | Tko | 5 | 13.27 |
|   |     | 20 | 12.63 |
|   |     | 50 | 13.91 |
|   |     | 100 | 16.78 |
|   |     | 200 | 21.68 |
|   |     | 300 | 14.52 |
|   |     | 400 | 3.18 |
|   |     | 500 | 2.14 |

As shown in FIGS. 12 and 13 and Table 9, a satisfactory S/N ratio was recognized when the Tris concentration in the reaction liquid was in the range of 300 mM or less (preferably, in the range of 5 mM or greater and 300 mM or less). Furthermore, the rise of the red fluorescence intensity was also rapid.

[Test Example 5: Application to digital measurement]

The efficiency for the detection of a target nucleic acid by means of digital measurement was evaluated by using the FEN proteins of Test No. 1 (abbreviated name Afu) and Test No. 6 (abbreviated name Tko).

A flat plate-shaped transparent substrate made of cycloolefin polymer (COP) having a plurality of microwells and a cover member made of COP were bonded together to produce a microfluidic device. A cover member having an inlet port and a discharge port so as to interpose the region where microwells were formed when the cover member was bonded, and having an elevated level difference of 50 $\mu$m at the peripheral edge part, was used. Each well of the microfluidic device was formed into a cylindrical shape. The diameter (opening diameter) of each well was designed to be 10 $\mu$m, and the depth of each well was designed to be 15 $\mu$m. The substrate and the cover member were produced by injection molding by using a thermoplastic resin. The edge part of the level difference was brought into contact with the substrate, and the contact portion was laser-welded.

As reagent 1, an aqueous solution including 150 aM target DNA oligo (manufactured by FASMAC Co., Ltd.: target nucleic acid: 5'-CCGAAGGGCATGAGCTGCATGATGAGCTGCACGGTGGAGGTG AGGCAGATGCCCAG-3': SEQ ID NO:8), 0.5 $\mu$M allele probe (manufactured by FASMAC Co., Ltd.: first nucleic acid: 5'-ACGGACGCGGAGTGCAGCTCATGCCC-3': SEQ ID NO:9), 1 $\mu$M invader oligo (manufactured by FASMAC Co., Ltd.: second nucleic acid: 5'-CCACCGTGCARCTCATCAA-3': SEQ ID NO:10), 4 $\mu$M FRET Cassette (RedmondRED-Eclipse Quanrure) (manufactured by Tsukuba Oligo Service Co., Ltd.: third nucleic acid and fourth nucleic acid: 5'-Redmond Red-TCT-Eclipse Quencher-TCGGCCTTTTGGCCGAGAGACTCCGCGTCCGT-3': SEQ ID NO: 11), FEN protein (abbreviated name Tko or abbreviated as Afu), 50 mM Tris-HCl (pH 8.5), 20 mM $MgCl_2$, and 0.05 v/v% Tween20 was prepared. Furthermore, a fluorine-based oil (FC40, manufactured by Sigma Aldrich Corp.) was used as an encapsulation liquid.

First, reagent 1 (20 $\mu$L) prepared as described above was delivered to the inlet port of the microfluidic device by using a pipette, and the microwells and the flow channels were filled with the reagent 1. Subsequently, a fluorine-based oil (150 $\mu$L) was delivered to the inlet port of the microfluidic device to replace the reagent 1 in the flow channel, and the reagent 1 was encapsulated in the microwells. Excess reagent 1 and fluorine-based oil were discharged through the discharge port.

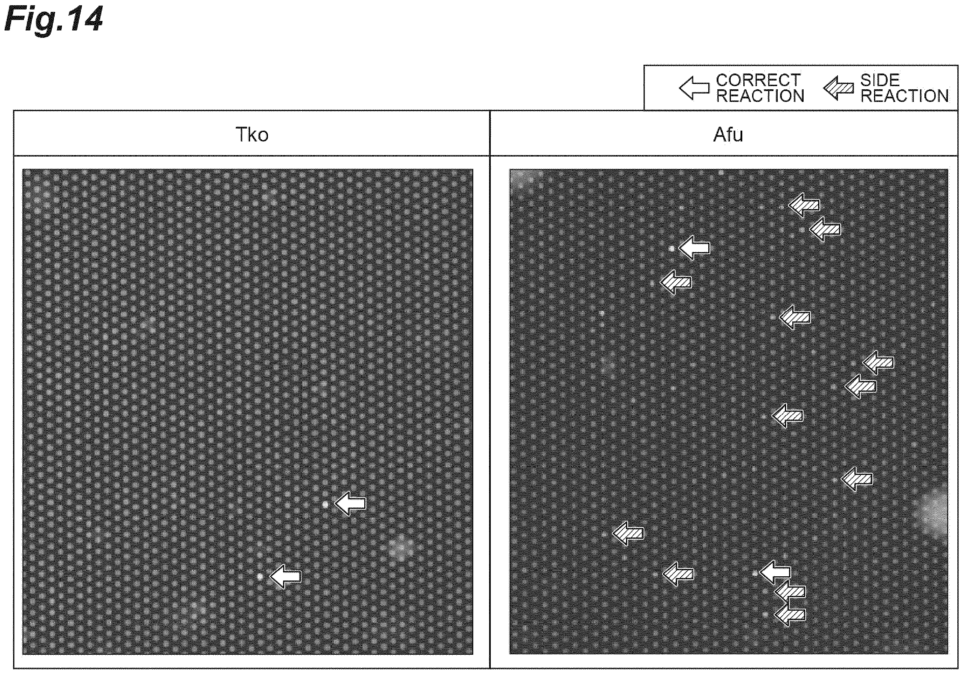

The microfluidic device was placed on a hot plate and heated at a hot plate temperature of 67°C, and a reaction was carried out for 25 minutes. Subsequently, observation of the microfluidic device was performed with a fluorescence microscope (BZ-X700, manufactured by Keyence CORPORATION.). The results are shown in FIG. 14.

From a preliminary experiment conducted in advance, the number of bright spots expected from the amount of input target nucleic acid (150 aM) was (1 well/13513 wells). As shown in FIG. 14, the results for the FEN protein of Test No. 6 (abbreviated name Tko) were almost consistent with the expected values. On the other hand, the results for the FEN protein of Test No. 1 (abbreviated name Afu) were significantly higher than the expected values, and most of them had dim brightness (side reaction).

**Claims**

1. A method for detecting a target nucleic acid, the method comprising:

   cleaving a first flap of a first cleavage structure formed by a target nucleic acid, a first nucleic acid, and a second nucleic acid;
   cleaving a second flap of a second cleavage structure formed by a third nucleic acid, the cleaved first flap, and a fourth nucleic acid; and
   detecting the presence of the target nucleic acid by detecting the cleaved second flap,
   wherein the cleaving the first flap and the cleaving the second flap are carried out by cleaving the first flap and the second flap with a flap endonuclease, and
   wherein the flap endonuclease has an amino acid sequence having a sequence identity of 96% or higher with the amino acid sequence of a flap endonuclease of a microbe selected from the group consisting of Thermococcus kodakarensis strain KOD1, Pyrococcus abyssi strain GE5, and Methanothermobacter thermautotrophicus strain Delta H.

2. The method according to claim 1, wherein the target nucleic acid is a DNA.

3. The method according to claim 1 or 2, wherein the cleaving the first flap and the cleaving the second flap are carried out under the conditions of a pH of 7.5 or higher and 9.0 or lower.

4. The method according to any one of claims 1 to 3, wherein the cleaving the first flap and the cleaving the second flap are carried out under the conditions of a temperature of 55°C or higher and 70°C or lower.

5. The method according to any one of claims 1 to 4, wherein the cleaving the first flap and the cleaving the second flap are carried out in an aqueous solvent, and the aqueous solvent includes $Mg^{2+}$.

6. The method according to claim 5, wherein a concentration of $Mg^{2+}$ in the aqueous solvent is 2.5 mM or greater and 20 mM or less.

7. The method according to any one of claims 1 to 6, wherein the cleaving the first flap and the cleaving the second flap are carried out in an aqueous solvent, and a concentration of $K^+$ in the aqueous solvent is 0 mM or greater and 100 mM or less.

8. The method according to any one of claims 1 to 7, wherein the cleaving the first flap and the cleaving the second flap are carried out in an aqueous solvent, and a concentration of trishydroxymethylaminomethane in the aqueous solvent is greater than 0 mM and 300 mM or less.

9. The method according to any one of claims 1 to 8, wherein the cleaving the first flap and the cleaving the second flap are carried out in an aqueous solvent, and a concentration of the flap endonuclease in the aqueous solvent is 0.5 $\mu$M or greater and 8 $\mu$M or less.

10. The method according to any one of claims 1 to 9, wherein the cleaving the first flap and the cleaving the second flap are carried out in an aqueous solvent, and a volume of the aqueous solvent is 10 aL or more and 10 $\mu$L or less.

11. The method according to any one of claims 1 to 10, wherein the third nucleic acid and the fourth nucleic acid are bonded by a linker molecule.

12. The method according to any one of claims 1 to 11, wherein the cleaved second flap is detected by detecting fluorescence.

13. A kit for use in the method according to any one of claims 1 to 12,
the kit comprising the third nucleic acid, the fourth nucleic acid, the flap endonuclease, and a manual indicating the guidelines for designing nucleotide sequences of the first nucleic acid and the second nucleic acid according to a nucleotide sequence of the target nucleic acid.

14. An expression vector comprising:

a nucleic acid sequence encoding an amino acid sequence having a sequence identity of 96% or higher with an amino acid sequence of a flap endonuclease of a microbe selected from the group consisting of Thermococcus kodakarensis strain KOD1, Pyrococcus abyssi strain GE5, and Methanothermobacter thermautotrophicus strain Delta H; and
one or a plurality of regulatory sequences operably linked to the nucleic acid sequence.

15. The expression vector according to claim 14, wherein the expression vector is a plasmid.

# Fig.1

FLAP

FEN

FIRST
NUCLEIC ACID

SECOND
NUCLEIC ACID

SECOND
REGION

FIRST
REGION

TARGET
NUCLEIC ACID

# Fig.2

EP 4 092 122 A1

# *Fig.3*

(A)

### Tko GREEN FLUORESCENCE

(B)

### Tko RED FLUORESCENCE

S/N=5.3

| | TARGET NUCLEIC ACID 0M |
| --- | --- |
| | TARGET NUCLEIC ACID 1.5pM |
| | TARGET NUCLEIC ACID 100nM |
| | TARGET NUCLEIC ACID 1 $\mu$M |

Fig.4

(A) Tko (pH7.5)

(B) Tko (pH8.5)

(C) Tko (pH8.0)

(D) Tko (pH9.0)

0M
100pM
1nM

# Fig.5

(A)

Tko (55℃)

(B)

Tko (60℃)

(C)

Tko (65℃)

(D)

Tko (67℃)

(E)

Tko (70℃)

0M
30pM

EP 4 092 122 A1

Fig.6

## Fig.7

**Fig.8**

(A)

Mth (55℃)

(B)

Mth (60℃)

(C)

Mth (65℃)

(D)

Mth (67℃)

(E)

Mth (70℃)

- - - - 0M

———— 1nM

Fig.9

(A) Tko (Mg²⁺ 1mM)

(B) Tko (Mg²⁺ 2.5mM)

(C) Tko (Mg²⁺ 5mM)

(D) Tko (Mg²⁺ 10mM)

(E) Tko (Mg²⁺ 20mM)

0M
100pM
1nM

Fig.10

(A)

Tko (K⁺ 0mM)

(B)

Tko (K⁺ 25mM)

(C)

Tko (K⁺ 50mM)

(D)

Tko (K⁺ 100mM)

(E)

Tko (K⁺ 200mM)

| | |
|---|---|
| | 0M |
| | 1nM |
| | 10nM |

Fig.11

(A) Mth (K⁺ 0mM)

(B) Mth (K⁺ 25mM)

(C) Mth (K⁺ 50mM)

(D) Mth (K⁺ 100mM)

(E) Mth (K⁺ 200mM)

0M
1nM
10nM

## Fig.12

# *Fig.13*

*Fig.14*

## INTERNATIONAL SEARCH REPORT

| International application No. |
| --- |
| PCT/JP2021/006198 |

**A. CLASSIFICATION OF SUBJECT MATTER**
C12N 15/55(2006.01)i; C12N 15/63(2006.01)i; C12Q 1/34(2006.01)i; C12Q 1/6813(2018.01)i
FI: C12N15/63 Z ZNA; C12N15/55; C12Q1/34; C12Q1/6813

According to International Patent Classification (IPC) or to both national classification and IPC

**B. FIELDS SEARCHED**

Minimum documentation searched (classification system followed by classification symbols)
C12N15/55; C12N15/63; C12Q1/34; C12Q1/6813

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/MEDUNE/EMBASE/BIOSIS (STN); GenBank/EMBL/DDBJ/GeneSeq; UniProt/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X | "Flap structure-specific endonuclease [Thermococcus kodakarensis KOD1]", GenBank: BAD85470.1 [online], 2018, retrieval date 14 April 2021, <https://www.ncbi.nlm.nih.gov/pratein/BAD85470.1>, entire text | 14, 15 |
| Y | entire text | 1-13 |
| X | "FEN1 FLAP endonuclease-1 [Pyrococcus abyssi GE5]", GenBank: CAB49654.1 [online], 2015, retrieval date 14 April 2021, <https://www.ncbi.nlm.nih.gov/pratein/CAB49654.1>, entire text | 14, 15 |
| Y | entire text | 1-13 |

☒ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 16 April 2021 (16.04.2021) | 27 April 2021 (27.04.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/006198 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X | "Flap endonuclease 1", UniProt: 027670 [online], 2003, retrieval date 14 April 2021, <https://www.uniprot.org/uniprot/027670.txt?version=117>, entire text | 14, 15 |
| Y | entire text | 1-13 |
| Y | WO 2019/026884 A1 (TOPPAN PRINTING CO., LTD.) 07 February 2019 (2019-02-07) claims 1, 3, 19, paragraphs [0003], [0042]-[0044], examples, etc. | 1-13 |
| Y | 牧野洋一他，生体分子のデジタル計測技術開発，日本印刷学会誌，2017, vol. 54, no. 6, pp. 377-382, section 4.2, fig. 6, (MAKINO, Yoichi et al., "Development of Digital Biomolecule Detection System", Journal of Printing Science and Technology) | 1-13 |
| Y | "Flap endonuclease-1 [Thermococcus gorgonarius]", NCBI Reference Sequence: WP_088884722.1 [online], 2017, retrieval date 14 April 2021, <https://www.ncbi.nlm.nih.gov/protein/1214773564?sat=49&satkey=24386714>, entire text | 1-13 |
| Y | "Flap endonuclease-1 [Pyrococcus horikoshii]", NCBI Reference Sequence: WP_010885498.1 [online], 2019, retrieval date 14 April 2021, <https://www.ncbi.nlm.nih.gov/protein/499187958?sat=49&satkey=24352886>, entire text | 1-13 |
| E, X | JP 6846763 B1 (TOPPAN PRINTING CO., LTD.) 24 March 2021 (2021-03-24) claims, examples, etc. | 1-15 |

Form PCT/ISA/210 (continuation of second sheet) (January 2015)

**INTERNATIONAL SEARCH REPORT**
Information on patent family members

| | | International application No. |
| --- | --- | --- |
| | | PCT/JP2021/006198 |

| Patent Documents referred in the Report | Publication Date | Patent Family | Publication Date |
| --- | --- | --- | --- |
| WO 2019/026884 A1 | 07 Feb. 2019 | EP 3663410 A1<br>claims 1, 3, 19,<br>paragraphs [0003],<br>[0056]-[0058],<br>examples<br>CN 110770355 A | |
| JP 6846763 B1 | 24 Mar. 2021 | (Family: none) | |

Form PCT/ISA/210 (patent family annex) (January 2015)

**REFERENCES CITED IN THE DESCRIPTION**

**Patent documents cited in the description**

- JP 2001518805 A

**Non-patent literature cited in the description**

- *Journal of Printing Science and Technology,* 2017, vol. 54 (6), 377-382